(19) Europäisches Patentamt
European Patent Office
Office européen des brevets

(11) EP 1 025 838 B1

(12) **EUROPEAN PATENT SPECIFICATION**

(45) Date of publication and mention of the grant of the patent: **16.04.2003 Bulletin 2003/16**

(51) Int Cl.[7]: **A61K 7/48**

(21) Application number: **99123365.1**

(22) Date of filing: **20.12.1996**

(54) **A cosmetic composition and a method for reducing the ageing processes of skin**

Kosmetikum und Verfahren zur Verzögerung des Alterns der Haut

Composition cosmétique et procédé de réduction des processus de vieillissement de la peau

(84) Designated Contracting States:
**AT BE CH DE DK ES FI FR GB GR IE IT LI LU MC NL PT SE**

(30) Priority: **22.12.1995 HU 9503728**

(43) Date of publication of application:
**09.08.2000 Bulletin 2000/32**

(62) Document number(s) of the earlier application(s) in accordance with Art. 76 EPC:
**96942539.6 / 0 869 771**

(73) Proprietor: **N-Gene Research Laboratories Inc.
New York, NY 10022 (US)**

(72) Inventors:
- **Literàti Nagi, Péter
1037 Budapest (HU)**
- **Farkas, Bea
6723 Szeged (HU)**
- **Vadàsz, Agnes
1037 Budapest (HU)**

(74) Representative: **von Füner, Nicolai, Dr. et al
v. Füner Ebbinghaus Finck Hano
Patentanwälte
Mariahilfplatz 2&3
81541 München (DE)**

(56) References cited:
**EP-A- 0 583 479**
**WO-A-90/04584**
**WO-A-97/16439**
**FR-A- 1 404 481**
**FR-A- 2 362 845**
**GB-A- 2 049 419**

Remarks:
The file contains technical information submitted after the application was filed and not included in this specification

EP 1 025 838 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The invention refers to the use of a hydroximic acid derivative of the formula

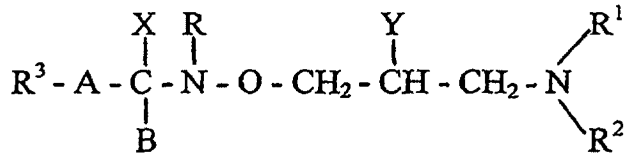

I

wherein

- $R^1$ represents a hydrogen atom or a $C_{1-5}$ alkyl group,
- $R^2$ stands for a hydrogen atom, a $C_{1-5}$ alkyl group, a $C_{5-7}$ cycloalkyl group or a phenyl group optionally substituted by a hydroxy or a phenyl group, or
- $R^1$ and $R^2$ together with the nitrogen atom they are attached to form a 5 to 8 membered ring optionally containing one or more further nitrogen or oxygen atom(s) and said ring can be condensed with a benzene ring,
- $R^3$ means a hydrogen atom, a phenyl group, a naphthyl group or a pyridyl group wherein said groups can be substituted by one or more halo atom(s) or $C_{1-4}$ alkoxy group(s),
- Y is a hydroxy group,
- X stands for an amino group,
- R forms with B a chemical bond,
- A is a group of the formula

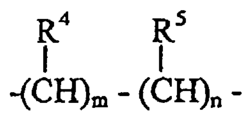

b

wherein

- $R^4$ represents a hydrogen atom,
- $R^5$ stands for a hydrogen atom,
- m has a value of 0, 1 or 2,
- n has a value of 0, 1 or 2,

or a physiologically acceptable acid addition salt thereof for the preparation of a composition for reducing ageing processes of the skin or treating pathological lesions of the skin. Furthermore, the invention refers to a cosmetic and a pharmaceutical composition comprising said hydroximic acid derivatives of the formula I.

The human skin is a natural target of light radiation having several known pleasant and unpleasant effects such as sunburn and carcinogenesis. Due to the ultraviolet radiation free radicals (for example hydroxy radicals or nascent oxygen) form in the skin. Such free radicals can injure the DNA and contribute to the ageing of the skin.

A well-known theory of the ageing process of skin is based on the deteriorating effect of free radicals. In addition to the effect of ultraviolet radiation, free radicals may also form in biochemical processes. Thus, due to e.g. inflammation, hypoxia or reactive hyperaemia, free radicals of oxygen origin such as superoxide anion, perhydroxy or hydroxy radical, hydrogen peroxide etc. may form.

Free radicals having powerful oxidizing effect can injure the membrane by oxidizing the unsaturated fatty acid components of the membrane (peroxidation of lipids) on the one hand, and reactive aldehydes are formed during the oxidization on the other hand In the injury of membrane, the increased intake of calcium leads to cell death, and pathological processes are started due to the presence of the reactive aldehydes:

- injury of DNA, mutation in both the cell nucleus and mitochondrium;
- change in the properties of the interstitial proteins (i.e. elastin) owing to formation of crosslinks.

It is known that the elastic structures of collagen proteins and elastin contain a lot of water. It is characteristic of the interstitial proteins that they are rich in lysine. The reactive aldehydes such as malondialdehyde give condensation reaction with the side chains containing amino groups to yield crosslinks. Thus, the originally elastic structure becomes rigid and hydrophobic. During the above process, at first lipofuscin ceroids, then age pigments are formed.

The natural protective mechanisms against ultraviolet radiation include bronzing due to the formation of melanin, DNA repair mechanism etc. The deficiency of a protective mechanism such as the damage of the DNA repair and consequently the loss of the correction of the DNA injuries caused by the ultraviolet rays leads to the early ageing of skin or perhaps to a disease called xeroderma pigmentosum that can be accompanied by the development of a malignant tumor. Sunburn spots caused by bronzing in the early childhood are healed leaving an extended scar. In addition to spinocellular carcinoma, various malignant tumors (e.g. melanoma, cerato-acanthoma, basalioma, sarcoma) can develop.

Thus, it is of great significance if the ageing processes of skin could be influenced and the pathological lesions could be treated.

The hydroximic acid derivatives of the formula I are known. HU-P No. 177 578 and its equivalent US-P No. 4,308,399 describe hydroximic acid derivatives within the compounds of the formula I suitable for the treatment of diabetic angiopathy.

HU-P No. 207 988 and its equivalent EP No. 417 210 also describe hydroximic acid halides having a selective beta-blocking effect, thus, being suitable for the treatment of diabetic angiopathy.

HU-P Application No. 2385/92 published under No. T/66350 describes further hydroximic acid derivatives. These known compounds can be used in the treatment of vascular deformations, mainly in the therapy of diabetes mellitus.

The aim of the invention is to provide a composition suitable for reducing the ageing processes of skin and/or treating pathological lesions of skin.

It was found that the above aim is fulfilled by a composition comprising a hydroximic acid derivative of the formula I or a physiologically acceptable acid addition salt thereof as the active ingredient.

Under the pathological lesions of skin for which the composition of the invention is suitable, especially the followings are meant:

- dry skin;
- actinic keratosis, aktinic prurigo (Lopez-Gonzalez's disease);
- polymorphic light exanthema;
- toxic photopathy;
- photo-allergy;
- purpura senilis;
- solar atrophy of skin;
- puberal strias (stria migrans);
- elastoma diffusum (old skin);
- X-ray dermatitis;
- gouty polychondritis;
- decubitus (bedsore).

In the description and Claims, under the term "composition" a composition is meant which is suitable in the first place for local treatment, and is applied to the skin surface in a conventional manner.

The composition of the invention comprises a hydroximic acid derivative of the formula I or a physiologically acceptable acid addition salt thereof as the active ingredient in admixture with one or more conventional carrier(s) of cosmetic compositions.

In the specification and Claims a $C_{1-5}$ alkyl group is, for example, a methyl, ethyl, n-propyl, isopropyl, n-butyl or n-pentyl group, preferably a methyl or an ethyl group.

A $C_{5-7}$ cycloalkyl group is cyclopentyl, cyclohexyl or cycloheptyl group, preferably a cyclopentyl or a cyclohexyl group.

A 5 to 8 membered ring containing one or more heteroatom(s) can be, for example, a pyrrole, pyrazole, imidazole, oxazole, pyridine, pyridazine, pyrimidine, piperazine, morpholine, indole, quinoline etc. ring.

A halo atom is, for example, a fluoro, chloro, bromo or iodo atom, preferably chloro or a bromo atom.

The physiologically acceptable acid addition salts of the compounds of the formula I are the acid addition salts formed with physiologically acceptable inorganic acids such as hydrochloric acid, sulfuric acid etc. or with physiologically acceptable organic acids such as acetic acid, fumaric acid, lactic acid etc.

A preferred subgroup of the compounds of the formula I consists of the hydroximic acid derivatives of the formula

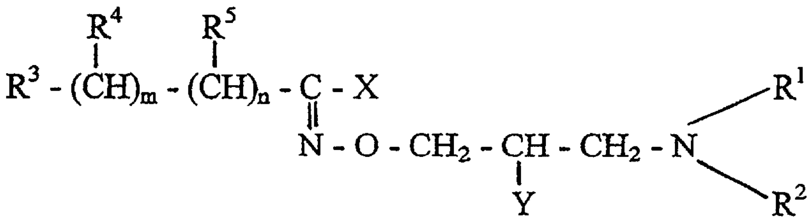

II

wherein $R^1$, $R^2$, $R^3$, $R^4$, $R^5$, m and n are as stated in relation to formula I, X represents an amino group, Y means a hydroxy group.

Especially preferred compounds of the formula II are those wherein $R^1$ and $R^2$ together with the nitrogen atom they are attached to form a piperidino group, $R^3$ stands for a pyridyl group, m and n have a value of 0, X is as defined above. Of these compounds, preferred species are as follows:

O-(3-piperidino-2-hydroxy-1-propyl)nicotinic amidoxime (Compound "B").

The compounds of the formula I can be prepared by the processes known from HU-P No. 177 578.

The composition of the invention contains, in general, 0.1 to 30 % by mass, suitably 2 to 10 % by mass, preferably 4 to 5 % by mass of a hydroximic acid derivative of the formula I or a physiologically acceptable acid addition salt thereof as the active ingredient and conventional carrier(s) of the cosmetic compositions.

The compositions of the invention can be conventional cosmetic formulations suitable for the local treatment of the skin surface. Preferred formulations are creams, body emulsions, sun-emulsions, skin treatment foams, sprays, skin regenerating ampouls etc.

The compositions of the invention may contain, in addition to the active ingredient the conventional carriers of cosmetic compositions generally in an amount of 70 to 99.9 % by mass. Suitable carriers are, for example, one- or two-basic alcohols having a saturated or an unsaturated carbon chain such as cetyl alcohol, stearyl alcohol, cetyl-stearyl alcohol, oleyl alcohol, lauryl alcohol, ethylene glycol, propylene glycol, glycerol; natural fats and oils such as olive oil, avocado oil, wheat-germ oil, maize-germ oil, lanolin, cocoa-butter; higher hydrocarbons such as vaseline oil, vaseline; bee-wax; cellulose derivatives; emulgators such as sodium lauryl sulfate, fatty acid or oleic acid esters of sorbitan, fatty acid or oleic acid esters of poly(ethylene glycol)s, sorbitan ethers of fatty alcohols or oleic alcohols, poly (ethylene glycol) ethers of fatty alcohols or oleic alcohols, glycerides of fatty acids; vitamins, herb extracts such as camomile extract; preservatives such as methyl, p-hydroxy-benzoate, chlorohexidine gluconate; light protecting factors etc.

The compositions of the invention are prepared by blending the ingredients thereof, in a manner known per se. In case of compositions based on a water/oil or oil/water emulsion, in general, the ingredients of the fatty phase and those of the aqueous phase are separately admixed, then the two phases are blended using a fatty phase of elevated temperature, if required. The active ingredient of the formula I is added, preferably in an aqueous solution, to the fatty phase or to the mixture of the other ingredients.

The skin ageing inhibition effect of the compounds of the formula I was examined on guinea-pigs. The skin surface of 8 guinea-pigs was depilated, then, on both sides of the animals 1 $cm^2$ areas were irradiated by UV-B light of 100 $mJ/cm^2$ intensity. After the irradiation one side of the animals was covered with the cream comprising 4 % by mass of the test compound as the active ingredient. The other side of each animal was covered with a mixture of cosmetic carriers comprising no active ingredient (i.e. a cream was used, which contained water instead of the active ingredient). Thus, as a matter of fact, an internal control was used in the experiment.

In case of 4 animals the treatment with the cream was performed immediately after the irradiation, then the treatment was repeated daily for a week (group I). In case of the other 4 animals the skin surface irradiated was treated with the cream of the invention and the control cream, respectively, only 24 hours after the irradiation (group II).

In case of animals of group I a minimal erythema could be observed on the skin surface irradiated and treated with the composition of the invention 24 and 48 hours after the irradiation. On the skin surface used as control an area without epithelium could be noticed, and this state subsisted during the 7 days of observation. From the 4th day no difference could be detected between the skin surface irradiated and treated with the composition of the invention and

the surrounding skin area.

In case of animals of group II, on the treated area as well as on the control area, skin injuries (vesicula, bulla) could be observed, then an area without epithelium developped. On the 7th day after the irradiation the area treated with the composition of the invention was epithelized.

On the basis of the above examination it can be established that the skin surface is protected from the damaging effects of the UV-B light by the composition of the invention and the compound of the formula I, respectively. If the skin surface is treated with the composition of the invention immediately after the irradiation, only a weak injury of the epithelium is experienced, at the most.

Skin injuries developped by UV-B radiation are healed owing to the treatment with the composition of the invention in a shorter time than without treatment. The compound of the formula I exerts an epithelizing effect.

Suitably, the skin surface is treated with a cosmetic composition comprising 0.1 to 30 % by mass of a hydroximic acid derivative of the formula I or a physiologically acceptable acid addition salt thereof.

Preferably, the skin surface is treated with O-(3-piperidino-2-hydroxy-1-propyl)nicotinic amidoxime or a physiologically acceptable acid addition salt thereof.

The invention is further elucidated by means of the following Examples.

**Example 1**

Moisturizing cream for night

The cream consists of the following ingredients:

| | |
|---|---|
| compound "B" | 5.0 % by mass |
| cetyl alcohol | 5.0 % by mass |
| lanolin (anhydrous) | 5.0 % by mass |
| cocoa-butter | 5.0 % by mass |
| vaseline | 5.0 % by mass |
| vaseline oil | 5.0 % by mass |
| isopropyl miristate | 1.0 % by mass |
| isopropyl palmitate | 1.0 % by mass |
| wheat-germ oil | 10.0 % by mass |
| evening primrose oil | 5.0 % by mass |
| vitamin A | 0.03 % by mass |
| vitamine E | 0.05 % by mass |
| glycerol | 5.0 % by mass |
| propylene glycol | 5.0 % by mass |
| methyl p-hydroxybenzoate | 0.2 % by mass |
| perfume | 0.1 % by mass |
| water, demineralized | 42.62 % by mass |
| | 100.00 % by mass |

**Example 2**

Moisturizing cream for day

The cream consists of the following ingredients:

| | |
|---|---|
| compound "B" | 5.0 % by mass |
| cetyl alcohol | 5.0 % by mass |
| lanolin (anhydrous) | 5.0 % by mass |
| vaseline | 5.0 % by mass |
| vaseline oil | 5.0 % by mass |
| isopropyl miristate | 1.0 % by mass |
| isopropyl palmitate | 1.0 % by mass |

(continued)

| | |
|---|---|
| borage oil | 4.0 % by mass |
| peanut oil | 11.0 % by mass |
| vitamin A | 0.03 % by mass |
| vitamin E | 0.05 % by mass |
| glycerol | 5.0 % by mass |
| propylene glycol | 5.0 % by mass |
| methyl p-hydroxybenzoate | 0.2 % by mass |
| perfume | 0.1 % by mass |
| water, demineralized | 47.62 % by mass |
| | 100.00 % by mass |

**Example 3**

Body milk

The body milk consists of the following ingredients :

| | |
|---|---|
| compound "B" | 4.0 % by mass |
| stearic acid monoglyceride | 2.0 % by mass |
| cetylstearyl alcohol | 2.0 % by mass |
| peanut oil | 5.0 % by mass |
| vaseline oil | 3.0% by mass |
| polyoxyethylene cetylstearyl alcohol (degree of polymerization: 20) | 2.0 % by mass |
| glycerol | 4.0 % by mass |
| methyl p-hydroxybenzoate | 0.2 % by mass |
| propyl p-hydroxybenzoate | 0.1 % by mass |
| butylhydroxytoluene | 0.01 % by mass |
| water, demineralized | 77.69 % by mass |
| | 100.00 % by mass |

**Claims**

**1.** Use of a hydroximic acid derivative of the formula

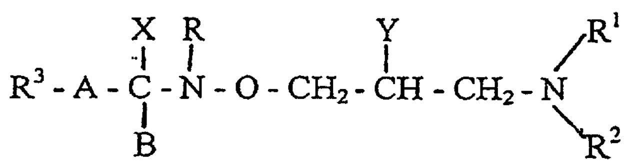

I

wherein

| | |
|---|---|
| $R^1$ | represents a hydrogen atom or a $C_{1-5}$ alkyl group, |
| $R^2$ | stands for a hydrogen atom, a $C_{1-5}$ alkyl group, a $C_{5-7}$ cycloalkyl group or a phenyl group optionally substituted by a hydroxy or a phenyl group, or |
| $R^1$ and $R^2$ | together with the nitrogen atom they are attached to form a 5 to 8 membered ring optionally containing one or more further nitrogen or oxygen atom(s) and said ring can be condensed with a benzene ring, |
| $R^3$ | means a hydrogen atom, a phenyl group, a naphthyl group or a pyridyl group wherein said groups can be substituted by one or more halo atom(s) or $C_{1-4}$ alkoxy group(s), |
| Y | is a hydroxy group, |
| X | stands for an amino group, |

R forms with B a chemical bond,
A is a group of the formula

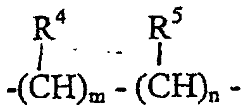 b

wherein

$R^4$ represents a hydrogen atom,

$R^5$ stands for a hydrogen atom,

m has a value of 0, 1 or 2,

n has a value of 0, 1 or 2,

or a physiologically acceptable acid addition salt thereof for the preparation of a composition for reducing ageing processes of the skin.

**2.** Use of the hydroximic acid derivative of the formula I as defined in claim 1 or the physiologically acceptable acid addition salt thereof for the preparation of a composition for treating pathological lesions of skin.

**3.** The use according to claim 1 or 2 in which the hydroximic acid derivative is a compound of the formula I, wherein $R^1$ and $R^2$ together with the nitrogen atom they are attached to form a piperidino group, $R^3$, A, X, B, R and Y are as defined in claim 1.

**4.** The use according to claim 3 in which the hydroximic acid derivative is O-(3-piperidino-2-hydroxy-l-propyl)nicotinic amidoxime.

**5.** A cosmetic composition comprising the hydroximic acid derivative of the formula I as defined in any of claims 1-4.

**6.** A pharmaceutical composition comprising the hydroximic acid derivative as defined in any of claims 1-4.

## Patentansprüche

**1.** Verwendung eines Isohydroxamsäurederivats der Formel

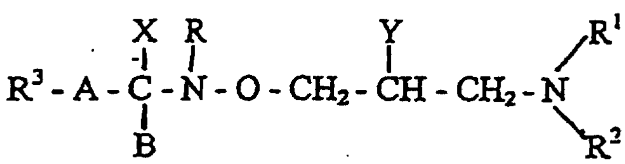 I

worin

$R^1$ ein Wasserstoffatom oder eine $C_{1-5}$-Alkylgruppe und
$R^2$ ein Wasserstoffatom, eine $C_{1-5}$-Alkylgruppe, eine $C_{5-7}$-Cycloalkylgruppe oder eine Phenylgruppe, die gegebenenfalls durch eine Hydroxy- oder eine Phenylgruppe substituiert sind, bedeuten oder
$R^1$ und $R^2$ zusammen mit dem Stickstoffatom, mit dem sie verknüpft sind, einen 5- bis 8-gliedrigen Ring bilden, der gegebenenfalls ein oder mehrere weitere(s) Stickstoff- oder Sauerstoffatom(e) enthält und mit einem Benzolring kondensiert sein kann,

$R^3$ ein Wasserstoffatom, eine Phenyl-, Naphthyl- oder Pyridylgruppe, wobei diese Gruppen durch ein oder mehrere Halogenatom(e) oder $C_{1-4}$-Alkoxygruppe(n) substituiert sein können,
Y eine Hydroxygruppe und
X eine Aminogruppe bedeuten,
R mit B eine chemische Bindung bildet und
A eine Gruppe der Formel

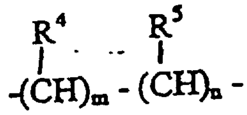

b

ist, worin

$R^4$ ein Wasserstoffatom,
$R^5$ ein Wasserstoffatom,
m einen Wert 0, 1 oder 2 und
n einen Wert 0, 1 oder 2 bedeuten,

oder eines physiologisch verträglichen Säureadditionssalzes davon für die Herstellung eines Gemisches zur Reduzierung von Alterungsprozessen der Haut.

2. Verwendung des Isohydroxamsäurederivats der Formel I nach Anspruch 1 oder des physiologisch verträglichen Säureadditionssalzes davon für die Herstellung eines Gemisches für die Behandlung pathologischer Hautläsionen.

3. Verwendung nach Anspruch 1 oder 2, bei der das Isohydroxamsäurederivat eine Verbindung der Formel I ist, worin $R^1$ und $R^2$ zusammen mit dem Stickstoffatom, mit dem sie verknüpft sind, eine Piperidinogruppe bilden, und $R^3$, A, X, B, R und Y wie in Anspruch 1 definiert sind.

4. Verwendung nach Anspruch 3, bei der das Isohydroxamsäurederivat 0- (Piperidino-2-hydroxy-1-propyl)nicotinamidoxim ist.

5. Kosmetisches Gemisch, das das Isohydroxamsäurederivat der Formel I nach einem der Ansprüche 1 bis 4 umfasst.

6. Pharmazeutisches Gemisch, das das Isohydroxamsäurederivat nach einem der Ansprüche 1 bis 4 umfasst.

## Revendications

1. Utilisation d'un dérivé de l'acide hydroximique répondant à la formule

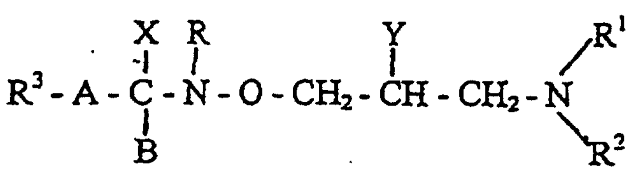

I

dans laquelle

$R^1$ représente un atome d'hydrogène ou un groupe alkyle en $C_1$-$C_5$;
$R^2$ représente un atome d'hydrogène, un groupe alkyle en $C_1$-$C_5$, un groupe cycloalkyle en $C_5$-$C_7$ ou un

$R^1$ et $R^2$ groupe phényle, le cas échéant substitué par un groupe hydroxyle ou par un groupe phényle, ou bien forment, ensemble avec l'atome d'azote auquel ils sont fixés, un noyau de 5 à 8 membres contenant le cas échéant un ou plusieurs atomes d'azote ou d'oxygène supplémentaires, ledit noyau pouvant être condensé à un noyau benzénique,

$R^3$ représente un atome d'hydrogène, un groupe phényle, un groupe naphtyle ou un groupe pyridyle, lesdits groupes pouvant être substitués par un ou plusieurs atomes d'halogène ou par un ou plusieurs groupes alcoxy en $C_1$-$C_4$,

Y représente un groupe hydroxyle,

X représente un groupe amino,

R forme avec B une liaison chimique,

A représente un groupe répondant à la formule

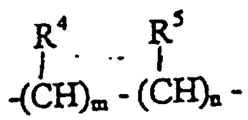

b

dans laquelle

$R^4$ représente un atome d'hydrogène,

$R^5$ représente un atome d'hydrogène,

m possède la valeur de 0,1 ou 2,

n possède la valeur de 0,1 ou 2,

ou d'un de ses sels d'addition d'acides physiologiquement acceptables pour la préparation d'une composition destinée à réduire les processus de vieillissement de la peau.

**2.** Utilisation du dérivé de l'acide hydroximique répondant à la formule I, tel que défini à la revendication 1, ou de son sel d'addition d'acide physiologiquement acceptable pour la préparation d'une composition destinée à traiter des lésions pathologiques de la peau.

**3.** Utilisation selon la revendication 1 ou 2, dans laquelle le dérivé de l'acide hydroximique est un composé répondant à la formule I, dans laquelle $R^1$ et $R^2$ forment, ensemble avec l'atome d'azote auquel ils sont fixés, un groupe pipéridino, $R^3$, A, X, B, R et Y sont tels que définis à la revendication 1.

**4.** Utilisation selon la revendication 3, dans laquelle le dérivé de l'acide hydroximique est l'amidoxime O-(3-pipéridino-2-hydroxy-1-propyl)nicotinique.

**5.** Composition cosmétique comprenant le dérivé de l'acide hydroximique répondant à la formule I tel que défini dans l'une quelconque des revendications 1 à 4.

**6.** Composition pharmaceutique comprenant le dérivé de l'acide hydroximique tel que défini dans l'une quelconque des revendications 1 à 4.